# EUROPEAN PATENT APPLICATION

(11) **EP 2 255 726 A1**
(43) Date of publication of application: **01.12.2010**
(21) Application number: 09290384.8
(22) Date of filing: 26.05.2009
(51) Int. Cl.: A61B 5/048

(54) **Spectral profile of SWS enhancing drugs**

(71) Applicant: Sanofi-Aventis, 75013 Paris (FR)
(72) Inventor: Coulouvrat, Catherine, 75013 Paris (FR)
(74) Representative: Morel-Pécheux, Muriel

(57) **Abstract**

Use of a spectral NREM (non-rapid eye movement) electroencephalographic (EEG) profile increasing the power density of 1 and 2 Hz bins and reducing power in beta frequency bins for identifying compounds displaying therapeutic activity on sleep disorders.

Compound with, in humans, a spectral NREM (non-rapid eye movement) electroencephalographic (EEG) profile increasing the power density of 1 and 2 Hz bins and reducing power,in beta frequency bins for sleep disorders.

## Description

The present invention relates to compounds having a spectral NREM (non-rapid eye movement) electroencephalographic (EEG) profile increasing the power density of 1 and 2 Hz bins and reducing power in beta frequency bins for sleep disorders.

The present invention also relates to the use of such spectral profile for identifying compounds displaying therapeutic activity on sleep disorders.

Compounds with a large variety of mechanisms of action with effect on SWS (slow wave sleep) and or SWA (slow wave activity) are known but all display side effects, such as no consistent improvement in sleep quality without inducing next-day sedation, or rebound or withdrawal symptoms after treatment discontinuation in patients with chronic insomnia and sleep maintenance difficulties.

It has been known for many years that neural pathways utilizing serotonin as their primary neurotransmitter play important roles in the control of sleep states. However, drugs which modulate serotoninergic transmission by, for example, stimulating or blocking receptors for this transmitter, have never found clinical use in the treatment of insomnia (Ann Pharm Fr 2007, vol. 65 pp. 268-274). This may soon change as several agents with selective activity as antagonists or inverse agonists at the 5HT_{2A} subtype of serotonin receptors are currently being tested in patients with sleep disorders. 5HT_{2A} antagonists do not show pharmacological effects similar to traditional sedative hypnotic agents. However, they do modify sleep architecture by selectively increasing slow wave sleep (Neuropsychopharmacology (1999); vol.21, pp. 455-466).

The compound (1Z, 2E)-1-(2-fluorophenyl)-3-(4-hydroxyphenyl)-prop-2-en-1-one-O-(2-dimethylaminoethyl)oxime, is hereafter referenced as eplivanserin or pharmaceutically acceptable salts or esters thereof. Documents EP 373998 and US 5166416 claim compounds having a generic scope encompassing eplivanserin or pharmaceutically acceptable salts or esters thereof and also specifically claimed eplivanserin or pharmaceutically acceptable salts or esters thereof, and disclosed its use as a platelet antiaggregant or a psychotropic agent.

See also US 6143792 and EP 1014960 (sleep apnea) and US 6576970 and EP 11140955 (snoring and upper airway resistance syndrome).

Eplivanserin or pharmaceutically acceptable salts or esters thereof, in particular hemifumarate salt, is an antagonist of 5HT_{2A} receptors (Journal of Pharmacological Experiment in Therapeutics, (1992), vol. 262 (2), pp. 759-68). Eplivanserin is well absorbed (>70%). Conventional dosage, between 1 and 10 mg, leads to a maximal plasma concentration that is reached between 2 and 6 hours; the half-life time of eplivanserin or pharmaceutically acceptable salts or esters thereof is relatively long, with an average value of 50 hours. Eplivanserin or pharmaceutically acceptable salts or esters thereof is also known to enhance slow wave sleep (SWS) (Neuropsychopharmacology (1999), vol.21 (3), pp. 455-466).

A clinical study was performed in order to define the spectral profile of eplivanserin and to examine the NREM EEG profile of eplivanserin in primary insomnia patients.

A polysomnographic (PSG) recording was performed on 102 patients aged of 18 years or more (52 women, 50 men) with primary insomnia. These patients were randomly selected from a group of approximately 300 patients with primary insomnia who received nightly eplivanserin 5mg during a six-week clinical trial.

Patients received placebo during two night-times and the screening/baseline with polysomnogram (PSG) was recorded. While receiving eplivanserin 5 mg on study days 41 and 42 PSG recordings were performed during two night-times.

The spectral analysis was conducted using a 4-second tapering window, overlapping 25%. This resulted in a ten 4-second windows, called sub-epochs, per 30-second epoch. The spectra from the sub-epochs were averaged across the 30-second epoch, and subsequently, all 30-second epochs were averaged together to get the spectral power for all NREM sleep during the entire night. The spectral power in quarter-Hz bins up to 31.5 Hz was calculated separately for each recording. The absolute power in 1 Hz bins up to 31 Hz was then calculated for each analyzed PSG recording by summing four 0.25 Hz bins (e.g. 0.25 + 0.50 Hz + 0.75 Hz + 1.0 Hz = 1 Hz band; 1.25 + 1.50 Hz + 1.75 Hz + 2.0 Hz = 2 Hz band). The absolute power values for the two screen/baseline PSGs were averaged, as were absolute power values for the two treatment nights.

The relative power was then calculated for each subject by dividing the average absolute power measures for the treatment nights by the absolute power values for the screen/baseline nights. A statistical comparison was made using t-tests with Bonferroni correction for multiple comparisons.

The absolute power in 1- and 2-Hz bins was significantly greater on eplivanserin treatment nights than at baseline (p<0.002), as were sub-delta (0.25-0.75 Hz) and delta (0.75-4.5 Hz) power (p<0.007). Therefore, eplivanserin shows a significantly lowered absolute power in 1-Hz bins from 12 to 23 Hz (p<0.002) and within the sigma (12-15 Hz), beta-1 (15-20 Hz), and beta-2 (20-25 Hz) bands (p<0.007) when compared with baseline.

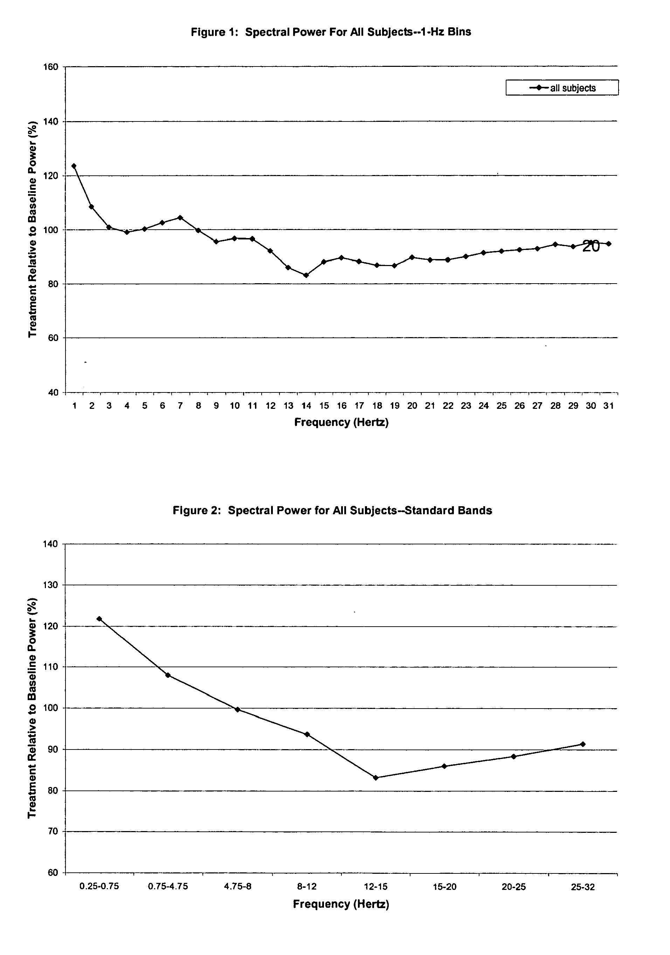

Figures 1 and 2 show spectral power in the 1-Hz bins and standard bands, respectively, for all patients; absolute power on treatment nights is expressed relative to baseline nights in these figures, for a better visual depiction of the data.

Similar analysis were performed on benzodiazepine (BzRA) and Tiagabine and provided different results. BzRAs reduced power in slow wave and theta frequencies, and tiagabine and other SWS-enhancing drugs increased power throughout the slow wave and theta bands.

These findings indicate that eplivanserin or pharmaceutically acceptable salts or esters thereof produces a NREM spectral profile that significantly differs from both hypnotics and other SWS-enhancing drugs. This spectral profile thus may be used to illustrate the distinct mechanism of action as compared to standard hypnotics and for identifying compounds displaying activity on sleep disorders, by enhancing SWS.

In some embodiments, sleep disorders are insomnia.

In some embodiments, sleep disorders are chronic insomnia.

In some embodiments, sleep disorders are insomnia characterized by sleep maintenance difficulties.

In some embodiments, sleep disorders are insomnia including nocturnal awakenings, usually for short-term duration.

In some embodiments, sleep disorders are insomnia including nocturnal awakenings or early awakenings, usually for short-term duration. In some embodiments, sleep disorders are chronic insomnia characterized by difficulties with sleep maintenance.

In some embodiments, a pharmaceutically acceptable salt of eplivanserin is hemifumarate.
Figure 1: Spectral power for all subjects -1 Hz Bins
Figure 2: Spectral power for all subjects-Standard bands

## Claims

1. Use of a spectral NREM (non-rapid eye movement) electroencephalographic (EEG) profile increasing the power density of 1 and 2 Hz bins and reducing power in beta frequency bins in humans for identifying compounds displaying therapeutic activity on sleep disorders.

2. Use according to claim 1, wherein said identified compound is a SWS-enhancing drug.

3. Use according to claim 1 or 2, wherein said identified compound is eplivanserin or pharmaceutically acceptable salts or esters thereof.

4. Use according to claims 1 to 3, wherein said identified compound is eplivanserin hemifumarate.

5. Use according to claims 1 to 4, wherein sleep disorders are insomnia.

6. Use according to claims 1 to 5, wherein sleep disorders are chronic insomnia.

7. Use according to claims 1 to 6, wherein sleep disorders are insomnia **characterized by** difficulties with sleep maintenance.

8. Use according to claims 1 to 7, wherein sleep disorders are chronic insomnia **characterized by** difficulties with sleep maintenance.

9. Compound with, in human, a spectral NREM (non-rapid eye movement) electroencephalographic (EEG) profile increasing the power density of 1 and 2 Hz bins and reducing power in beta frequency bins for sleep disorders.

10. Compound according to claim 9, wherein said compound is eplivanserin or pharmaceutically acceptable salts or esters thereof.

11. Compound according to claim 8 or 10, wherein said compound is eplivanserin hemifumarate.

12. Compound according to claims 8 to 11, wherein sleep disorders are insomnia.

13. Compound according to claims 8 to 12, wherein sleep disorders are chronic insomnia.

14. Compound according to claims 8 to 13, wherein sleep disorders are insomnia **characterized by** difficulties with sleep maintenance.

15. Compound according to claims 8 to 14, wherein sleep disorders are chronic insomnia **characterized by** difficulties with sleep maintenance.

16. Compound according to claims 8 to 15, wherein a therapeutic amount of eplivanserin is 5 mg once a day.

17. Compound according to claims 8 to 16, wherein the administration can be made at any time of the day.
